Europäisches Patentamt

European Patent Office

Office européen des brevets

(11) Publication number: **0 390 106**
**A2**

(12) **EUROPEAN PATENT APPLICATION**

(21) Application number: **90105909.7**

(22) Date of filing: **28.03.90**

(51) Int. Cl.⁵: **A61M 1/00**

(30) Priority: **31.03.89 JP 82469/89**

(43) Date of publication of application:
**03.10.90 Bulletin 90/40**

(84) Designated Contracting States:
**BE DE FR GB IT SE**

(71) Applicant: **Terumo Kabushiki Kaisha**
**No. 44-1, Hatagaya 2-chome Shibuya-ku**
**Tokyo 151(JP)**

(72) Inventor: **Sugisawa, Yasuhiko**
**c/o Terumo K.K., 150, Maimaigi-cho**
**Fujinomiya-shi, Shizuoka-ken(JP)**
Inventor: **Takeoka, Norio**
**c/o Terumo K.K., 150, Maimaigi-cho**
**Fujinomiya-shi, Shizuoka-ken(JP)**

(74) Representative: **Casalonga, Axel et al**
**BUREAU D.A. CASALONGA - JOSSE**
**Morassistrasse 8**
**D-8000 München 5(DE)**

(54) **Fluid aspirator.**

(57) A fluid aspirator (1) which withdraws a body fluid by suction has a resiliently expandable and compressible suction casing (2) for collecting the withdrawn body fluid. A check valve (13) is mounted in a hollow plug (7) which is detachably disposed in a communication port (5) that is defined in the suction casing (2). The check valve (13) discharges air from the suction casing (2) when the suction casing (2) is compressed. After the suction casing (2) is compressed, the check valve (13) is closed to maintain a partial vacuum developed in the suction casing (2). The check valve (13) comprises a valve member in the shape of a disc or a ball which is made of a hard, inflexible material.

## FIG.1

EP 0 390 106 A2

## BACKGROUND OF THE INVENTION

The present invention relates to a fluid aspirator for slowly and continuously withdrawing a fluid such as blood, an exudate, or the like from a patient's body.

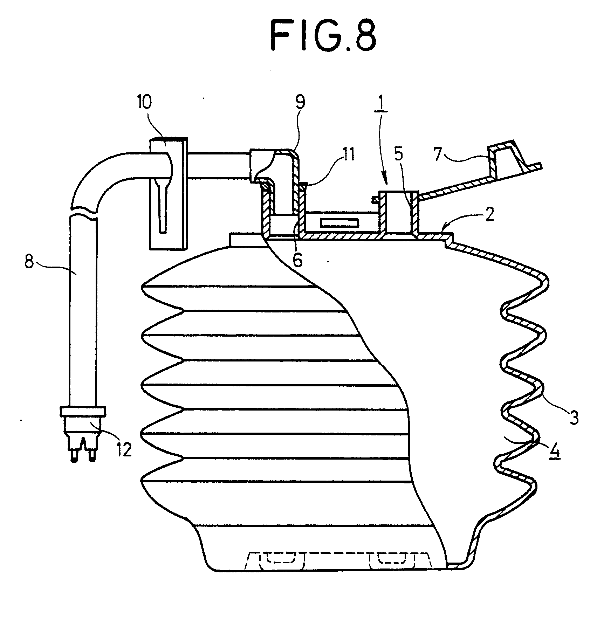

FIG. 8 of the accompanying drawings shows one conventional fluid aspirator 1'. The fluid aspirator 1' has a suction casing 2 comprising bellows 3 which is resiliently expandable of its own accord from its contracted state, thereby changing the volume of the inner space 4 of the bellows 3. The suction casing 2 has a communication port 5 and a suction port 6, through which the inner space 4 communicates with the exterior space. The communication port 5 is openably closed by a detachable hollow plug 7. A suction tube 8 is connected through an elbow tube 9 to the suction port 6. A clamp 10 is attached to the suction tube 8. The elbow tube 9, which is inserted at one end into the suction port 6, is sealed by a gasket 11 for leakage-free communication with the suction port 6. A connector 12 for connection to a suction catheter or the like is attached to the free end of the suction tube 8.

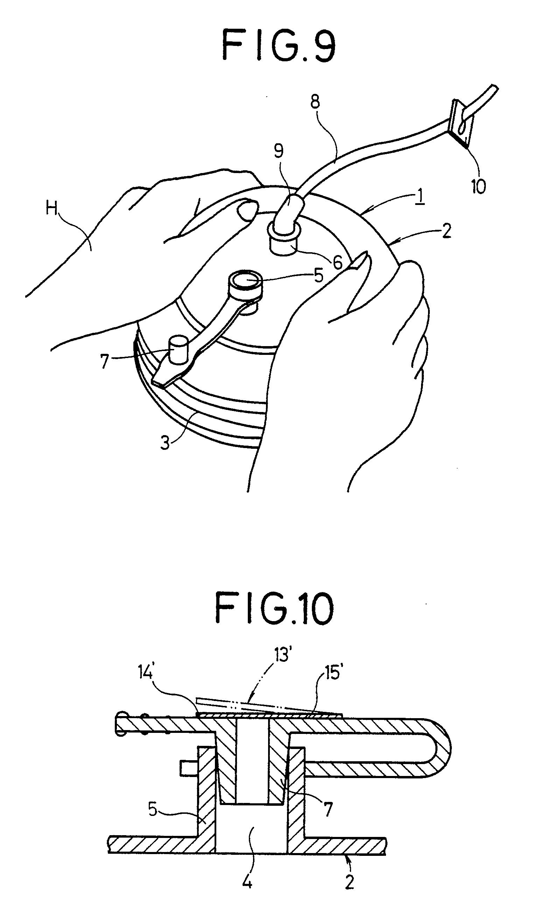

In use, as shown in FIG. 9, the hollow plug 7 is detached from the communication port 5, and the suction tube 8 is closed off by the clamp 10. Then, the suction casing 2 is compressed with hands H to discharge air from the inner space 4 out of the communication port 5. After the suction casing 2 has been compressed, the hollow plug 7 is fitted into the communication port 5, isolating the inner space 4 out of communication with the exterior space. Thereafter, the hands H are released from the suction casing 2. Since no air flows into and out of the suction casing 2, the suction casing 2 remains compressed. At this time, a partial vacuum is developed within the suction casing 2 owing to the self-recovering resiliency of the suction casing 2. A suction catheter or the like which is inserted into a desired region of the body of a patient is coupled to the suction tube 8 through the connector 12, so that the suction aspirator 1' is connected to the region of the patient's body where a body fluid is to be removed. Thereafter, the clamp 10 is released to open the suction tube 8. Now, the body fluid is withdrawn from the body region through the suction tube 8 and the suction port 6 into the suction casing 2 under the self-recovering resiliency of the bellows 3 which tends to recover the original shape of the bellows 3.

With the conventional fluid aspirator 1', in order to compress the suction casing 2 against the self-recovering resiliency of the bellows 3 to create a partial vacuum in the suction casing 2, it is necessary to remove the hollow plug 7 from the communication port 5, then compress the suction casing 2 from its expanded state, and insert the hollow plug 7 again into the communication port 5 while the suction casing 2 is being compressed. Since only one hand is used to keep the suction casing 2 compressed, it is relatively difficult to develop a partial vacuum in the suction casing 2. Inasmuch as the other hand is occupied to insert the hollow plug 7 into the communication port 5, the suction casing 2 is liable to become somewhat expanded under its own resiliency before the hollow plug 7 is fully inserted in the communication port 5. When this happens, the actual capacity of the suction casing 2 for withdrawing the body fluid becomes smaller than the potential capacity of the suction casing 2.

In an effort to solve the above problem, the applicant has developed a medical aspirator container as disclosed in Japanese Patent Publication No. 63(1988)-26656. The disclosed aspirator container is essentially of the same construction as the fluid aspirator shown in FIGS. 8 and 9, except that a check valve 13' is mounted on the communication port 5 as shown in FIG. 10. More specifically, the hollow plug 7 has a through hole, and the check valve 13' comprises a valve membrane 14' of a soft material mounted on the hollow plug 7 over one end of the through hole therein. In use, the hollow plug 7 remains fitted in the communication port 5. When the suction casing 2 is compressed, the check valve 13' is opened as indicated by the two-dot-and-dash line in FIG. 10. When the suction casing 2 is expanded to its original shape, the check valve 13' is closed as indicated by the solid line. Therefore, after the suction casing 2 has been compressed, it is not necessary to insert the hollow plug 7 into the communication port 5. A partial vacuum is quickly developed in the suction casing 2 immediately after the suction casing 2 has been compressed. The process of creating a partial vacuum in the suction casing 2 is relatively easy. As the hollow plug 7 is not required to be inserted into the communication port 5 after the manual compression of the suction casing 2, both hands can be used to keep the suction casing 2 compressed, without the effective capacity of the suction casing 2 for body fluid withdrawal being reduced.

One problem with the check valve 13' is that the valve membrane 14' tends to stick to a valve seat 15' of the check valve 13'. When the valve membrane 14' remains firmly attached to the valve seat 15', it is not opened when the suction casing 2 is to be compressed, and the suction casing 2 cannot be compressed. If the suction casing 2 were forcibly compressed at this time, the valve membrane 14' might be ruptured under an excessive pressure buildup in the suction casing 2. In order to eliminate this drawback, the user has to check the valve membrane 14′, and peel it off the valve seat 15′ before the suction casing 2 can be compressed. Consequently, the process of compressing the suction casing 2 is not easy and foolproof with the proposed aspirator container.

## SUMMARY OF THE INVENTION

It is a primary object of the present invention to provide a fluid aspirator which can easily be compressed, is free from a check valve breakdown, allows a partial vacuum to be developed easily in a suction casing, and can keep a maximum effective capacity for body fluid withdrawal.

Another object of the present invention is to provide a fluid aspirator comprising a suction casing having an expandable and compressible inner space defined therein and resiliently recoverable from a compressed state to an expanded state, a suction tube, a suction port defined in the suction casing and providing fluid communication between the inner space and an exterior space, the suction port being connected to the suction tube, for withdrawing a fluid through the suction tube into the inner space in response to recovery thereof from the compressed state to the expanded state, a communication port defined in the suction casing, for discharging air from the inner space in response to compression of the suction casing from the expanded state to the compressed state, and a check valve disposed in the communication port, the check valve comprising a valve housing which has a lower valve seat and an upper valve holder, and a valve member placed on and movable away from the lower valve seat toward the upper valve holder, whereby the valve member is lifted off the valve seat, thereby opening the check valve, when the suction casing is compressed from the expanded state to the compressed state, and the valve member is seated on the valve seat, thereby closing the check valve, when the suction casing is expanded from the compressed sate to the expanded state.

Still another object of the present invention is to provide the fluid aspirator further including a hollow plug detachably mounted in the communication port, the check valve being mounted in the hollow plug.

Yet another object of the present invention is to provide the fluid aspirator wherein the valve member is hard and inflexible.

A further object of the present invention is to provide the fluid aspirator wherein the valve member is in the shape of a disc.

A still further object of the present invention is to provide the fluid aspirator wherein the valve member is in the shape of a ball.

The above and other objects, features and advantages of the present invention will become more apparent from the following description when taken in conjunction with the accompanying drawings in which preferred embodiments of the present invention are shown by way of illustrative example.

## BRIEF DESCRIPTION OF THE DRAWINGS

FIG. 1 is a side elevational view, partly in cross section, of a fluid aspirator according to an embodiment of the present invention;

FIG. 2 is an enlarged cross-sectional view of a check valve mounted in a plug of the fluid aspirator shown in FIG. 1;

FIG. 3 is a plan view of a suction catheter to be used with the fluid aspirator shown in FIG. 1;

FIGS. 4 and 5 are perspective views showing the manner in which the fluid aspirator shown in FIG. 1 is used;

FIGS. 6(a), 6(b), and 6(c) are enlarged cross-sectional views showing the manner in which the check valve operates;

FIG. 7 is an enlarged cross-sectional view of a check valve according to another embodiment of the present invention;

FIG. 8 is a side elevational view, partly in cross section, of a conventional fluid aspirator;

FIG. 9 is a perspective view showing the manner in which the conventional fluid aspirator is used; and

FIG. 10 is an enlarged cross-sectional view of a conventional check valve.

## DETAILED DESCRIPTION OF THE PREFERRED EMBODIMENTS

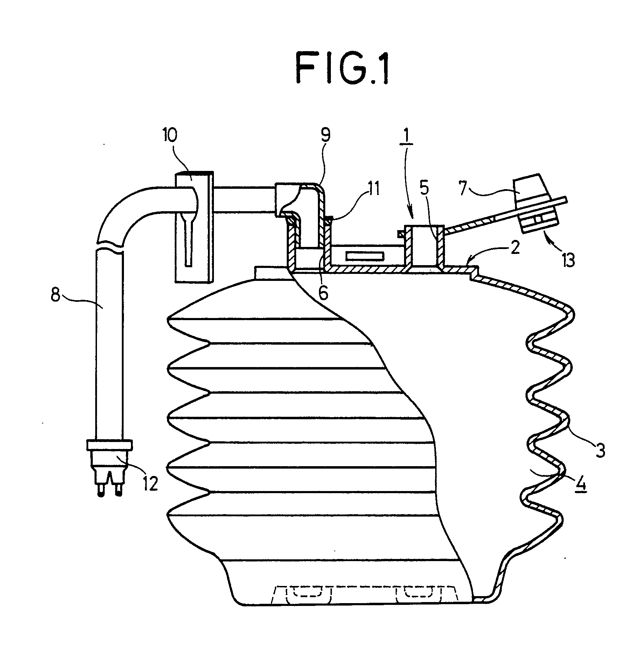

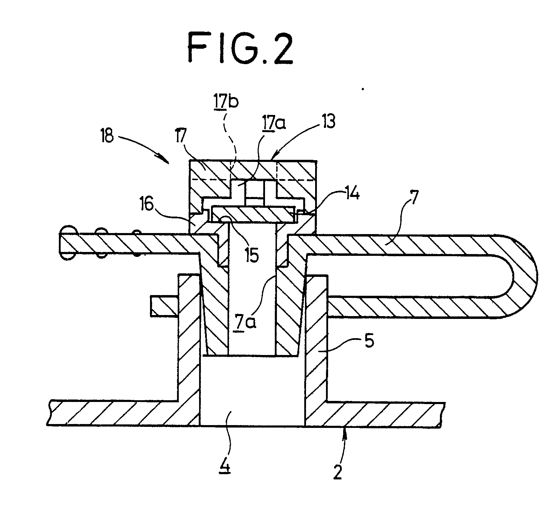

As shown in FIGS. 1 and 2, a fluid aspirator 1 according to an embodiment of the present invention is of essentially the same construction as that of the conventional fluid aspirator 1′ shown in FIGS. 8 and 9. Those parts shown in FIGS. 1 and 2 which are identical to those shown in FIGS. 8, 9, and 10 are denoted by identical reference numerals, and will not be described in detail.

The fluid aspirator 1 according to the present invention differs from the fluid aspirator 1′ shown in FIGS. 8, 9, and 10 with respect to a check valve 13 mounted on the hollow plug 7 detachably mounted in the communication port 5.

The communication port 5 is defined in an upper wall of the suction casing 2 of the fluid aspirator 1, and the hollow plug 7 is detachably mounted in the communication port 5. The hollow plug 7 has a through hole 7a defined therethrough. The check valve 13 according to the present invention is fixedly fitted in the through hole 7a. The check valve 13 comprises a inflexible disc-shaped valve member 14 made of a hard synthetic resin. The valve member 14 is placed on, but movable away from, a lower valve seat 15 on the upper surface of a valve seat ring 16 which is fitted in the through hole 7a in the hollow plug 7. An upper valve holder 17 for holding the valve member 14 is fixedly mounted on the upper surface of the valve seat ring 16. The valve seat ring 16 and the valve holder 17 jointly constitute a valve housing 18. The valve holder 17 has radial and axial vent holes 17a, 17b which allows easy escape of air from the valve housing 18 when the valve member 14 is lifted off the valve seat 15.

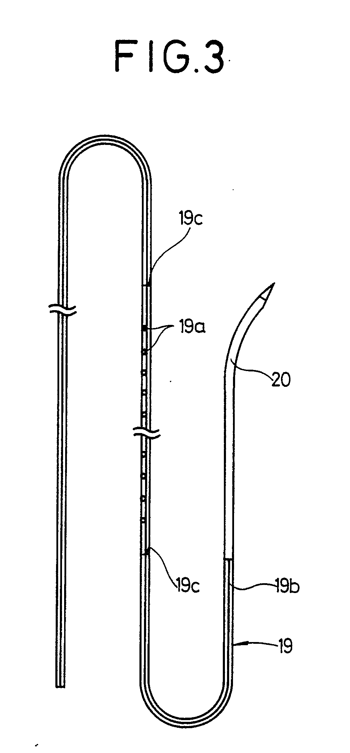

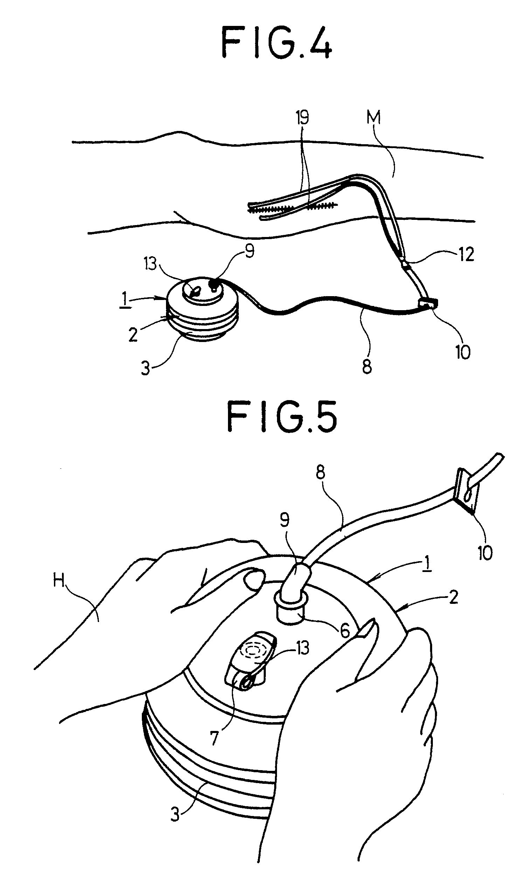

The fluid aspirator 1 is connected to a suction catheter 19 shown in FIG. 3. More specifically, the suction catheter 19 is coupled at one end thereof to the suction tube 8 through the connector 12. The suction catheter 19 includes a cannula 20 on its other end for insertion into the body of a patient. The catheter 19 also has a number of side holes 19a defined in a side wall thereof. An X-ray contrast line 19b for indicating the position of the suction catheter 19 in an X-ray photograph is attached to the suction catheter 19. Stop lines 19c for confirming the position of the suction catheter 19 in the patient's body are also applied to the suction catheter 19. As shown in FIG. 4, two suction catheters 19 are connected to the suction tube 8 and inserted in the patient's body, denoted at M, so that the side holes 19a are positioned within a desired region, such as a wound, of the patient's body M.

The fluid aspirator 1 thus constructed will be used as follows:

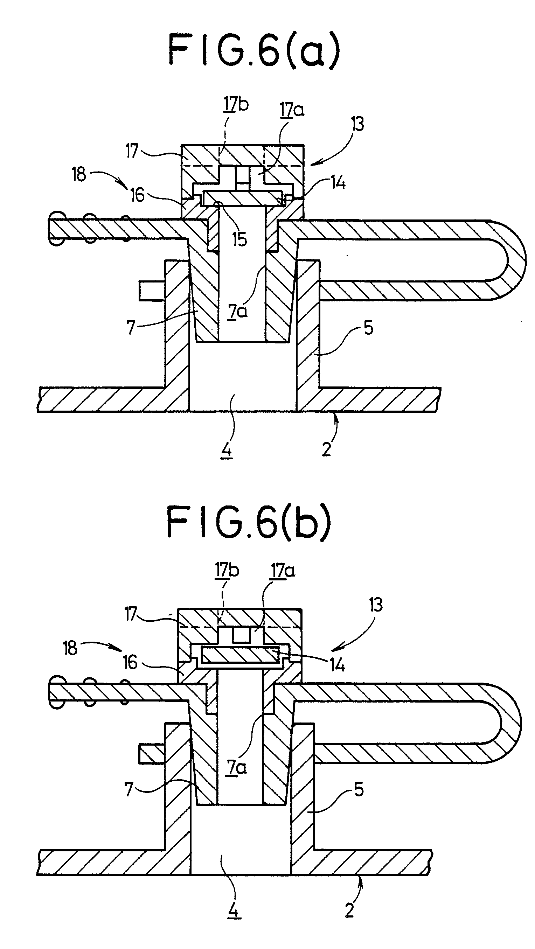

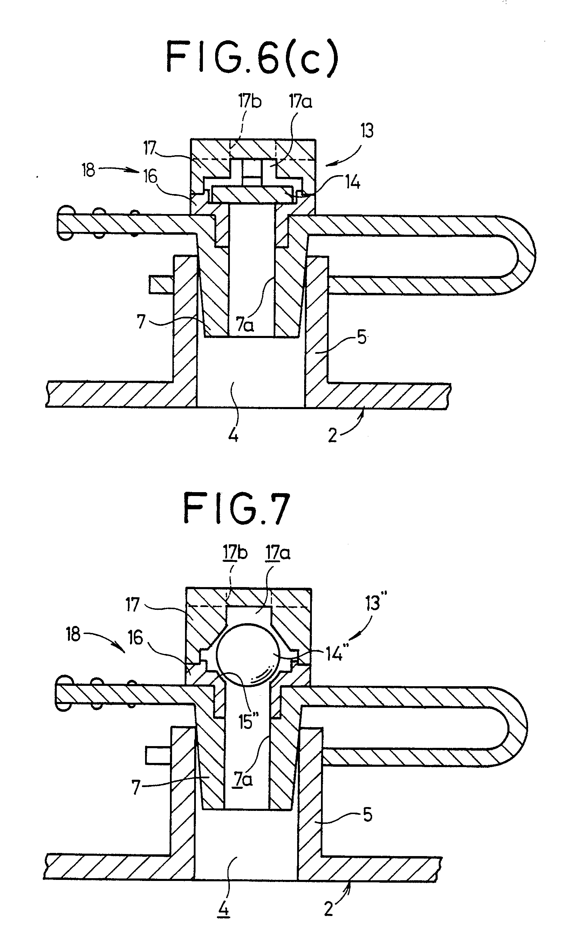

As shown in FIG. 5, the hollow plug 7 is fitted into the communication port 5 of the fluid aspirator 1, and the suction tube 8 is closed off by the clamp 8. Then, the suction casing 2 is resiliently compressed by hands H. Since air in the inner space 4 in the suction casing 2 is compressed, the air pressure in the suction casing 2 is increased (see FIG. 6(a)). Therefore, the valve member 14 of the check valve 13 is lifted off the valve seat 15 of the valve seat ring 16 under the air pressure buildup, as shown in FIG. 6(b). The air in the inner space 4 is now discharged out of the vent holes 17a, 17b in the valve holder 17.

After the air is discharged from the inner space 4, since a partial vacuum is developed in the suction casing 2, the valve member 14 is seated on the valve seat 15, thereby closing the hole 7a, as shown in FIG. 6(c). Accordingly, no air is introduced into the suction casing 2 even if the heads H are released from the suction casing 2. The fluid aspirator 1 now remains compressed without the aid of the hands H, and the partial vacuum is maintained in the suction casing 2 owing the self-recovering resiliency of the bellows 3. The suction catheters 19 are connected to the connector 12 coupled to the suction tube 8, and inserted in the patient's body M so that the side holes 19a are positioned within a desired body region, such as a wound.

Thereafter, the clamp 10 is released to open the suction tube 8. A body fluid such as blood or an exudate is slowly and continuously withdrawn from the wound of the patient's body under the vacuum developed by the self-recovering resiliency of the bellows 3. The amount of the withdrawn fluid may be measured according to graduations marked on the suction casing 2. To discharge the collected fluid from the suction casing 2, the fluid remaining in the suction tube 8 is first drained, then the suction tube 8 is closed off by the clamp 10, thereafter the hollow plug 7 is removed, and the fluid aspirator 1 is turned upside down so that the communication hole 5 is directed downwardly to discharge the collected fluid.

FIG. 7 shows a check valve 13″ according to another embodiment of the present invention. The check valve 13″ comprises a ball valve member 14″ and the valve seat ring 16 has a partly spherical valve seat 15″ for receiving the ball valve member 14″. The other structural details shown in FIG. 7 are substantially the same as those shown in FIG. 2.

With the present invention, as described above, the fluid aspirator includes a check valve which comprises a valve housing having a lower valve seat and an upper valve holder, and a valve member placed on and movable away from the valve seat. The check valve is disposed in the communication port of the suction casing. When the suction casing is resiliently compressed, the valve member is lifted off the valve seat, thereby opening the check valve. When the suction casing is expanded, the valve member is seated on the valve seat, thereby closing the check valve. Therefore, a vacuum can easily be created in the suction casing, and the effective capacity of the suction casing for fluid withdrawal is not reduced. In addition, since the valve member does not stick to the valve seat, the suction casing can also easily be compressed, and the valve member is prevented from being damaged or broken down. The check valve is therefore durable in operation.

The check valve is mounted in the hollow plug which is disposed in the communication port. Therefore, the communication port is used not only to discharge air from the inner space in the suction casing, but also to drain a fluid collected in the suction casing. Consequently, the fluid aspirator is simple in construction.

Since the valve member is made of a hard, inflexible material, it does not stick to the valve seat and is not susceptible to damage or breakdown. The fluid aspirator is therefore easy to use and high in durability.

Although certain preferred embodiments have been shown and described, it should be understood that many changes and modifications may be made therein without departing from the scope of the appended claims.

## Claims

1. A fluid aspirator comprising:
a suction casing having an expandable and compressible inner space defined therein and resiliently recoverable from a compressed state to an expanded state;
a suction tube;
a suction port defined in said suction casing and providing fluid communication between said inner space and an exterior space, said suction port being connected to said suction tube, for withdrawing a fluid through said suction tube into said inner space in response to recovery thereof from the compressed state to the expanded state;
a communication port defined in said suction casing, for discharging air from said inner space in response to compression of said suction casing from the expanded state to the compressed state; and
a check valve disposed in said communication port, said check valve comprising a valve housing which has a lower valve seat and an upper valve holder, and a valve member placed on and movable away from said lower valve seat toward said upper valve holder, whereby said valve member is lifted off said valve seat, thereby opening said check valve, when said suction casing is compressed from the expanded state to the compressed state, and said valve member is seated on said valve seat, thereby closing said check valve, when said suction casing is expanded from the compressed sate to the expanded state.

2. A fluid aspirator according to claim 1, further including a hollow plug detachably mounted in said communication port, said check valve being mounted in said hollow plug.

3. A fluid aspirator according to claim 1 or 2, wherein said valve member is hard and inflexible.

4. A fluid aspirator according to claim 3, wherein said valve member is in the shape of a disc.

5. A fluid aspirator according to claim 3, wherein said valve member is in the shape of a

ball.

# FIG.1

EP 0 390 106 A2

# FIG.2

# FIG.3

# FIG.4

# FIG.5

# FIG.6(a)

# FIG.6(b)

EP 0 390 106 A2

# FIG.6(c)

# FIG.7

# FIG.8

# FIG.9

# FIG.10